# NEUE EUROPÄISCHE PATENTSCHRIFT

(11) **EP 2 755 018 B2**
(45) Veröffentlichungstag und Bekanntmachung des Hinweises auf die Entscheidung über den Einspruch: **03.04.2024**
(45) Hinweis auf die Patenterteilung: 22.07.2020
(21) Anmeldenummer: 13151359.0
(22) Anmeldetag: 15.01.2013
(51) Int. Cl.: G01N 21/94, G01N 21/3563, A22C 21/00, A22C 25/04, G01N 21/84, A22C 25/18, G01N 33/12, G01B 11/25, A22C 17/00

(54) **Vorrichtung und Verfahren zur berührungslosen Erkennung roter Gewebestrukturen sowie Anordnung zum Lösen eines Streifens roter Gewebestrukturen**
Device and method for the non-contact detection of red tissue structures and assembly for detaching a strip of red tissue structures
Dispositif et procédé de reconnaissance sans contact de structures tissulaires rouges ainsi qu'agencement de dissolution d'une bande de structure tissulaire rouge

(43) Veröffentlichungstag der Anmeldung: 16.07.2014
(73) Patentinhaber: Nordischer Maschinenbau Rud. Baader GmbH + Co. KG, 23560 Lübeck (DE)
(72) Erfinder: Grimm, Oliver, 19061 Schwerin (DE); Rünger, Björn, 23562 Lübeck (DE); Irmler, Arne, 23562 Lübeck (DE)
(74) Vertreter: Stork Bamberger Patentanwälte PartmbB

(56) Entgegenhaltungen:
- WO-A1-01/09587
- WO-A1-89/12397
- WO-A1-89/12397
- WO-A1-2008/016309
- WO-A1-2008/016309
- GB-A- 2 264 602
- US-A- 3 800 363
- US-A- 3 800 363
- US-A- 4 557 019
- US-A- 5 324 228
- US-A- 5 324 228
- US-A- 5 471 311
- US-B1- 6 369 401

## Beschreibung

Die vorliegende Erfindung betrifft eine Vorrichtung zur berührungslosen Erkennung roter Gewebestrukturen in Produkten geschlachteter Tierkörper. Des Weiteren betrifft die vorliegende Erfindung ein Verfahren zum berührungslosen Erkennen roter Gewebestrukturen in Produkten geschlachteter Tierkörper sowie eine Anordnung zum Lösen eines Streifens roter Gewebestrukturen aus Produkten geschlachteter Tierkörper.

Solche Vorrichtungen, Verfahren und Anordnungen kommen in unterschiedlichen Bereichen der industriellen Verarbeitung von geschlachteten Tierkörpern, beispielsweise bei der Verarbeitung von Fisch-, Fleisch- oder Geflügelprodukten zum Einsatz, insbesondere bei der Verarbeitung von Fischfilets, beispielsweise bei der Verarbeitung von Thunfischen, z.B. von Skipjacks. Bei den roten Gewebestrukturen handelt es sich in der Regel um stark durchblutete Gewebetypen, die sich optisch von anderen Geweben unterscheiden. Beispielsweise umfassen die genannten roten Gewebestrukturen stark durchblutetes Muskelfleisch.

Es ist bekannt, die zu untersuchenden Produkte, beispielsweise Fischfilets, mit Licht zu beaufschlagen und die reflektierte Teilstrahlung bzw. die in dem Produkt, insbesondere in einem transluzenten Produkt gestreute Lichtstrahlung, mittels einer Detektionseinrichtung zu detektieren, um Rückschlüsse auf die Produktbeschaffenheit zu ziehen. Eine Vorrichtung sowie ein Verfahren zum kontaktlosen Erkennen von Charakteristika transluzenter Produkte geht beispielsweise aus dem Dokument DE 10 2008 013 525 B4 hervor. Die WO 89/12397 A1 offenbart ein Verfahren zur Erkennung vom Fleischgewebe, insbesondere in Fisch.

Die US 3,800,363 offenbart eine Vorrichtung und ein Verfahren zum Schlachten vom Thunfisch.

Die US 5,324,228 befasst sich mit einem Verfahren und eine Vorrichtung zum Beschneiden von Fett in Fleischprodukten.

Mit den bekannten Vorrichtungen und Verfahren ist es zwar möglich, Fremdkörper und/oder Fremdgewebetypen zu detektieren, jedoch findet keine Differenzierung zwischen verschiedenen Gewebetypen statt, so dass sich die bekannten Vorrichtungen und Verfahren nicht zur Erkennung von bestimmten Gewebetypen, insbesondere nicht zur Erkennung stark durchbluteter Gewebe eignen.

Es ist daher Aufgabe der vorliegenden Erfindung, eine Vorrichtung vorzuschlagen, die eine zuverlässige Erkennung und Differenzierung unterschiedlicher durchbluteter Gewebetypen in den Produkten gewährleistet. Des Weiteren besteht die Aufgabe darin, ein entsprechendes Verfahren vorzuschlagen. Ferner besteht die Aufgabe darin, eine Anordnung vorzuschlagen, die das automatische Herauslösen bestimmter Gewebetypen aus den Produkten zuverlässig gewährleistet.

Die Aufgabe wird durch eine Vorrichtung mit den Merkmalen des Anspruchs 1 gelöst. Das verwendete infrarote Licht wird in den Bereichen der roten Gewebestrukturen stärker in dem Produkt absorbiert als dies in den übrigen Gewebebestandteilen des Produkts der Fall ist. Der Unterschied fällt besonders groß zwischen den roten Gewebestrukturen und dem übrigen Gewebe aus, so dass die Bereiche mit den roten Gewebestrukturen exakt und mit hoher Zuverlässigkeit erkannt werden. Die vorliegende Erfindung eignet sich daher grundsätzlich zur Erkennung von roten Gewebestrukturen, beispielsweise von stark durchbluteten Geweben, insbesondere von rotem Muskelfleisch. Auch ist es möglich, verletzte Gewebebereiche, beispielsweise hämatöse Bereiche, von anderen Gewebestrukturen bzw. Produktbereichen zu differenzieren.

Nach der Erfindung sind die Lichtquelle und das optische Sensormittel in Förderrichtung voneinander beabstandet angeordnet. Aufgrund der daraus resultierenden Anordnung der Lichtquelle und des optischen Sensormittels bezüglich des Produktes ist es möglich, zugleich ein Höhenprofil bzw. eine Höhenkontur des Produktes zu erfassen.

Gemäß der Erfindung ist die Detektionseinrichtung derart angeordnet, dass die von dem Produkt reflektierten Lichtanteile unter einem Betrachtungswinkel kleiner 90° gegenüber der Förderrichtung aufgenommen werden. Dies bietet den Vorteil, dass die quer zur Förderrichtung auf dem Produkt verlaufende Lichtlinie nicht aufgrund von Oberflächenunebenheiten des Produktes teilweise verschattet wird. So wird die Lichtlinie von der Detektionseinrichtung lückenlos bzw. unterbrechungsfrei und weitestgehend unabhängig von Unebenheiten der Produktoberfläche erfasst.

Gemäß der Erfindung sind außerdem die Lichtquelle und die Detektionseinrichtung derart angeordnet, dass der Lichtfeldwinkel größer als der Betrachtungswinkel ist. Dies bietet den Vorteil, dass die reflektierten Lichtanteile, die von der Detektionseinrichtung unter dem Betrachtungswinkel aufgenommen werden nicht durch Unebenheiten auf der Oberfläche des Produktes verschattet oder verdeckt werden können, so dass die reflektierten Lichtanteile in jedem Fall ungehindert zu der Detektionseinrichtung gelangen.

Dadurch, dass die Detektionseinrichtung der Lichtquelle in Förderrichtung nachgeordnet angeordnet ist, wird ebenfalls gewährleistet, dass die Lichtlinie auf dem Produkt durchgängig und unabhängig von Oberflächenunebenheiten aus dem flächigen Lichtfeld gebildet wird.

Gemäß einer weiteren bevorzugten Ausführungsform ist die Lichtquelle als Linienlaser ausgebildet. Der Linienlaser bietet den Vorteil, dass das Produkt einerseits mit Licht hoher Intensität beaufschlagt wird, so dass das Licht entsprechend weit in das Produkt eindringt. So ist eine Erkennung bzw. Differenzierung von tieferliegenden Gewebestrukturen, beispielsweise Muskelfleischbereichen, möglich. Anderseits ist das Licht des Linienlasers stark fokussiert, so dass die Lichtlinie auf dem Produkt möglichst schmal ausgebildet ist. Mit anderen Worten wird mit dem Linienlaser eine starke Fokussierung des Lichtstroms in einem möglichst schmalen flächigen Lichtfeld erzielt, so dass die Lichtlinie auf dem Produkt sowohl mit hoher Intensität als auch stark fokussiert auftrifft. Dies bietet den Vorteil einer hohen Ortsauflösung bei der Erkennung der roten Gewebestrukturen sowie einer sehr guten Empfindlichkeit bei der Differenzierung zwischen den roten Gewebestrukturen und anderen Gewebetypen und/oder weiteren Bestandteilen des Produktes.

Erfindungsgemäß weist die Lichtquelle eine Wellenlänge zwischen 650 nm und 900 nm auf. Licht mit einer Wellenlänge zwischen 650 nm und 900 nm wird aufgrund der besonders ausgeprägten Absorptionseigenschaften der roten Gewebestrukturen stark absorbiert, so dass die in dem Produkt gestreuten bzw. reflektierten Lichtanteile entsprechend stark gedämpft werden. Aus diesem Grund weist die von der Detektionseinrichtung ausgewertete Linienbreite auf dem Produkt in Bereichen roter Gewebestrukturen eine deutlich geringere Breite auf, als in anderen Bereichen, da die starke Dämpfung des Lichtstromes in den roten Gewebestrukturen den Lichtstreubereich entsprechend stark reduziert. In den übrigen Bereichen, in denen die Absorption des einfallenden Lichtstromes deutlich geringer ist, ist der Streubereich deutlich größer, so dass die Linienbreite der Lichtlinie in diesen Bereichen entsprechend breiter ist. Auf Basis der verschiedenen Lichtlinienbreiten ist so eine zuverlässige und exakte Erkennung der roten Gewebestrukturen möglich.

Gemäß einer weiteren bevorzugten Ausbildung der Erfindung ist die Detektionseinrichtung zum Aufnehmen mehrerer Einzelbilder ausgebildet und eingerichtet. Mit anderen Worten ist die Detektionseinrichtung zum getakteten Aufnehmen eine Vielzahl von Bildern von dem Produkt bzw. von der auf dem Produkt vorhandenen Lichtlinie ausgebildet und eingerichtet. Auf diese Weise wird der Verlauf der Kontur des Produktes erfasst, so dass die Position der roten Gewebestrukturen über die gesamte Produktlänge ermittelt wird.

Eine weitere zweckmäßige Ausbildung der Erfindung ist dadurch gekennzeichnet, dass die Detektionseinrichtung ein Auswertemittel umfasst, das zum Bestimmen von Grenzlinien zwischen Bereichen der roten Gewebestrukturen und den übrigen Produktbereichen des Produktes auf Basis der Einzelbilder ausgebildet und eingerichtet ist. Dies bietet den Vorteil, dass die Detektionseinrichtung zur Ermittlung der Grenzen zwischen den roten Gewebestrukturen und den übrigen Bereichen ausgebildet und eingerichtet ist. Die Grenzlinien können beispielsweise für eine nachfolgende Bearbeitung des Produktes als Schnittlinien dienen.

Gemäß einer weiteren vorteilhaften Ausbildung der Erfindung ist das Auswertemittel zum Ermitteln von Linien gleicher Lichtintensität in den Einzelbildern zur Bestimmung der Grenzlinien ausgebildet und eingerichtet. Die Lichtlinien, bzw. die Linienverläufe gleicher Lichtintensität, die als Isoluxe bezeichnet werden, bilden so die Ausgangsbasis zur Bestimmung der Grenzlinien. Auf diese Weise ist es ferner möglich, durch Vorgabe eines Lichtintensitätsschwellwertes den von den Grenzlinien begrenzten Bereich um die roten Gewebestrukturen variabel einzustellen. Mittels des Lichtintensitätsschwellenwertes wird so vorgegeben, an welcher Position bzw. in welchem Abstand von den roten Gewebestrukturen die Grenzlinien detektiert bzw. ermittelt werden. Mit anderen Worten kann so ein Toleranzbereich eingestellt werden, um zu gewährleisten, dass keine Bestandteile der roten Gewebestrukturen außerhalb des von den Grenzlinien bezeichneten Bereichs in dem Produkt vorhanden sind.

Des Weiteren wird die Aufgabe durch eine Anordnung mit den Merkmalen des Anspruchs 7 gelöst. Mittels der erfindungsgemäßen Anordnung ist es erstmalig möglich, die roten Gewebestrukturen vollständig automatisiert aus den Produkten zuverlässig und exakt herauszulösen.

Gemäß einer weiteren bevorzugten Ausführungsform ist die Schneideinrichtung relativ zu dem Produkt bewegbar ausgebildet. Dies bietet den Vorteil, dass die Schneideinrichtung während des Vorbeilaufens eines der kontinuierlich geförderten Produkte an das Produkt herangeführt, bzw. in das Produkteingeführt werden kann, um so die Schnittführung exakt an die Bereiche der ermittelten roten Gewebestrukturen bzw. an die Grenzlinien anzupassen.

Eine weitere zweckmäßige Ausgestaltung der Erfindung ist dadurch gekennzeichnet, dass die Schneideinrichtung mindestens ein Stellglied umfasst, mittels dessen die Schneideinrichtung zum teilweisen oder vollständigen Lösen der roten Gewebestrukturen aus dem Produkt steuerbar eingerichtet ist. Mittels des Stellgliedes ist die Position der Schneiderichtung mittels der Steuerungseinrichtung einstellbar ausgebildet, so dass das Lösen der roten Gewebestrukturen vollständig automatisch und im Idealfall ohne eine manuelle Nachbearbeitung erfolgt.

Gemäß einer weiteren bevorzugten Ausbildung der Erfindung ist die Schneideinrichtung höhenverstellbar ausgebildet und eingerichtet, so dass zum teilweisen oder vollständigen Lösen der roten Gewebestrukturen aus dem Produkt die Eintauchtiefe der Schneideinrichtung in das Produkt veränderbar ausgebildet ist. Mittels der veränderbar eingerichteten Eintauchtiefe, also einem Maß, wie weit beispielsweise eine Schneidkante der Schneideinrichtung in das Produkt eindringt, wird das Höhenprofil des Produktes beim Lösen der roten Gewebestrukturen berücksichtigt, so dass eine vollständige Trennung bzw. ein teilweises Lösen der roten Gewebestrukturen von dem restlichen Produkt mit hoher Präzision erfolgt.

Die vorliegende Aufgabe wird auch durch ein Verfahren mit den Merkmalen des Anspruchs 11 gelöst. Das erfindungsgemäße Verfahren bietet -wie schon zuvor im Zusammenhang mit der erfindungsgemäßen Vorrichtung im Detail erläutert -den Vorteil einer zuverlässigen und exakten Erkennung der roten Gewebestrukturen in den Produkten.

Das Aufnehmen der von dem Produkt reflektierten Lichtanteile erfolgt unter einem Betrachtungswinkel kleiner 90° gegenüber der Förderrichtung. Dies bietet den Vorteil, dass die quer zur Förderrichtung auf dem Produkt verlaufende Lichtlinie nicht aufgrund von Oberflächenunebenheiten des Produkts teilweise verschattet wird. So wird die Lichtlinie von der Detektionseinrichtung lückenlos bzw. unterbrechungsfrei und weitestgehend unabhängig von Unebenheiten der Produktoberfläche erfasst.

Der Lichtfeldwinkel ist größer als der Betrachtungswinkel. Dies bietet den Vorteil, dass die reflektierten Lichtanteile, die von der Detektionseinrichtung unter dem Betrachtungswinkel aufgenommen werden nicht durch Unebenheiten auf der Oberfläche des Produktes verschattet oder verdeckt werden können, so dass die reflektierten Lichtanteile in jedem Fall ungehindert zu der Detektionseinrichtung gelangen.

Eine weitere zweckmäßige Ausbildung der Erfindung ist dadurch gekennzeichnet, dass mehrere Einzelbilder mittels der Detektionseinrichtung von jedem der Produkte aufgenommen werden. Mittels der Aufnahme mehrerer Einzelbilder von dem Produkt bzw. von der Lichtlinie auf dem Produkt wird der Verlauf der Kontur des Produktes erfasst, so dass die Position der roten Gewebestrukturen über die gesamte Produktlänge ermittelt wird.

Eine weitere zweckmäßige Ausgestaltung der Erfindung ist dadurch gekennzeichnet, dass auf Basis der Einzelbilder mittels eines Auswertemittel Grenzlinien zwischen Bereichen der roten Gewebestrukturen und den übrigen Produktbereichen des Produktes bestimmt werden.

Gemäß einer weiteren vorteilhaften Ausgestaltung der Erfindung werden Linien gleicher Lichtintensität in den Einzelbildern zur Bestimmung der Grenzlinien mittels der Auswertemittel bestimmt. Die jeweiligen Vorteile wurden bereits zuvor im Zusammenhang mit der erfindungsgemäßen Vorrichtung beschrieben, so dass zur Vermeidung von Wiederholungen auf die entsprechenden Passagen der Beschreibung verwiesen wird.

Weitere bevorzugte und/oder zweckmäßige Merkmale und Ausgestaltungen der Erfindung ergeben sich aus den Unteransprüchen und der Beschreibung. Besonders bevorzugte Ausführungsformen werden anhand der beigefügten Zeichnung näher erläutert. In der Zeichnung zeigt:
- Fig. 1: eine schematische Darstellung einer ersten Ausführungsform der erfindungsgemäßen Vorrichtung in Seitenansicht,
- Fig. 2: eine schematische Darstellung eines Querschnitts eines Thunfisches,
- Fig. 3: eine schematische Darstellung eines Thunfischfilets einschließlich eines Teils einer Fördereinrichtung in perspektivischer Darstellung und
- Fig. 4: eine schematische Darstellung des in Fig. 3 dargestellten Thunfischfilets sowie des Teils der Förderreinrichtung im Querschnitt.

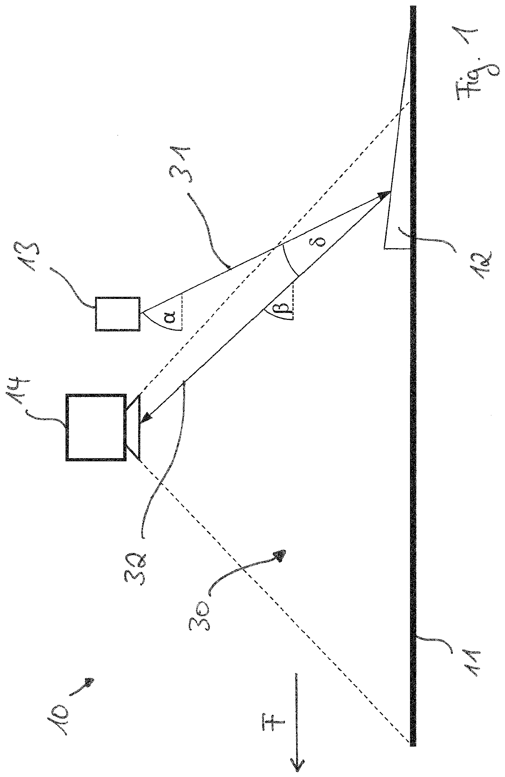

In der Fig. 1 ist eine schematische Darstellung einer ersten Ausführungsform der erfindungsgemäßen Vorrichtung 10 in Seitenansicht gezeigt. Die Vorrichtung 10 umfasst eine Fördereinrichtung 11, mittels derer die Produkte 12 in eine Förderrichtung F kontinuierlich gefördert werden. Beispielsweise ist die Fördereinrichtung 11 als Endlosförderer ausgebildet. Die Produkte 12 sind vorzugsweise Teile von geschlachteten Tierkörpern, beispielsweise Fisch-, Fleisch- oder Geflügelteile. Besonders bevorzugt sind die Produkte 12 Fischfilets von Thunfischen.

Die Vorrichtung 10 umfasst ferner eine Lichtquelle 13. Die Lichtquelle 13 ist derart eingerichtet und ausgebildet, dass diese ein flächiges Lichtfeld 31 aussendet, wobei das flächige Lichtfeld 31 eine quer zu der Förderrichtung F verlaufende Lichtlinie bildet. Anders ausgedrückt ist die Lichtquelle 13 zur Bildung einer Lichtlinie auf dem Produkt 12, die quer zu der Förderrichtung F verläuft, ausgebildet und eingerichtet.

Die Vorrichtung 10 umfasst weiter eine Detektionseinrichtung 14 mit einem optischen Sensormittel. Alternativ umfasst die Detektionseinrichtung 14 mehrere Sensormittel. Das optische Sensormittel ist zur Aufnahme der von dem Produkt 12 reflektierten Lichtanteile (32) ausgebildet und eingerichtet. Das optische Sensormittel ist beispielsweise als CCD-Kamera oder als CCD-Zeilenkamera ausgebildet. Vorzugweise ist die Lichtquelle 13 eine Infrarotlichtquelle. Besonders bevorzugt ist die Lichtquelle 13 zur Aussendung von Licht im infraroten Wellenlängenbereich ausgebildet. Alternativ umfasst die Lichtquelle 13 einen Wellenlängenbereich ausgehend vom Infrarotbereich bis hin zum sichtbaren Rot.

Die Lichtquelle 13 ist derart angeordnet, dass die Ebene des flächigen Lichtfeldes 31 gegenüber der Förderrichtung F um einen Lichtfeldwinkel α kleiner 90° geneigt ist. Mit anderen Worten ist die Ebene vorzugsweise nicht orthogonal zu der Förderrichtung F ausgerichtet, so dass die Ebene des flächigen Lichtfeldes 31 nicht senkrecht auf die Oberfläche der Produkte 12 trifft, sondern um einen entsprechenden Winkelbetrag geneigt ist. Die Schrägstellung des flächigen Lichtfeldes 31 führt zu einer Verformung der Lichtlinien auf dem Produkt 12 in Abhängigkeit der Geometrie des Produktes 12, so dass anhand des Verlaufs der Lichtlinien auf die Form und Geometrie des Produktes 12 rückgeschlossen werden kann.

Vorzugsweise ist das optische Sensormittel der Detektionseinrichtung 14 zur Aufnahme von Bildern der entsprechend der Geometrie des Produktes 12 verlaufenden Lichtlinien ausgebildet und eingerichtet und auf den jeweiligen Wellenlängen des von der Lichtquelle 13 ausgesandten Lichts angepasst.

Alternativ kann die Detektionseinrichtung 14 auch weitere optische Sensoren umfassen, die zur Aufnahme von Bildern bzw. von Bilddaten in einem anderen, beispielsweise im sichtbaren, Wellenlängenbereich ausgebildet und eingerichtet sind. Die Detektionseinrichtung 14 ist in diesem Fall zur Verarbeitung der Signale mehrerer der optischen Sensoren eingerichtet. Beispielsweise weist die Detektionseinrichtung 14 Verarbeitungsmittel zur Überlagerung der aufgenommenen Bilder von mehreren der optischen Sensoren auf.

Des Weiteren ist zusätzlich zu der Lichtquelle 13 eine weitere - in der Zeichnung nicht gezeigte - Lichtquelle zur Beleuchtung des Produktes 12 mit Licht sichtbarer Wellenlänge angeordnet. Während die Detektionseinrichtung 14 neben dem optischen Sensormittel zur Aufnahme von Bildern des Produktes 12 und der auf diesem verlaufenden Lichtlinien im Infrarotbereich umfasst, ist zusätzlich ein weiteres - in der Zeichnung ebenfalls nicht gezeigtes - optisches Sensormittel zur Aufnahme von Bildern des Produktes 12 im sichtbaren Lichtwellenlängenbereich angeordnet. Besonders bevorzugt sind das optische Sensormittel sowie das weitere optische Sensormittel als ein integrales Sensormittel, d.h. zusammen als ein Sensormittel, ausgebildet. Beispielsweise ist dieses eine Sensormittel als Kamera ausgebildet, die sowohl für den infraroten als auch für den sichtbaren Lichtwellenlängenbereich empfindlich ist. Mit anderen Worten umfasst die Detektionseinrichtung 14 vorzugsweise nur ein Sensormittel, das sowohl zur Aufnahme von Bildern im infraroten als auch im sichtbaren Lichtwellenlängenbereich ausgebildet und eingerichtet ist. Wie zuvor beschrieben ist die Detektionseinrichtung 14 vorzugsweise dazu angepasst, mittels des Verarbeitungsmittels durch Kombination der aufgenommenen Bilder im sichtbaren sowie im infraroten Lichtwellenlängenbereich zusätzliche Informationen über die Beschaffenheit, insbesondere der Geometrie, der Produkte 12 zu ermitteln. Beispielsweise ist es auf diese Weise möglich, geometrische Daten des Produktes 12, wie die Produktbreite oder die Produktkontur zu ermitteln.

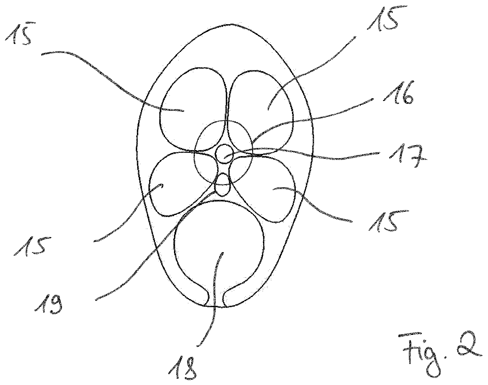

Anhand der Figur 2, die eine schematische Darstellung eines Querschnitts eines Thunfisches zeigt, soll die Funktionsweise der erfindungsgemäßen Vorrichtung zur berührungslosen Erkennung der roten Gewebestrukturen 16 beispielhaft erläutert werden. Bei den in der Figur 2 gezeigten roten Gewebestrukturen 16 handelt es sich um rotes Muskelfleisch. Die vorliegende Erfindung ist jedoch nicht auf die Erkennung des roten Muskelfleisches 16 bei Thunfischen beschränkt, sondern eignet sich grundsätzlich auch für andere Produkte 12, also beispielsweise für Filets oder Fleischbestandteile anderer Tierarten.

Der in der Figur 2 gezeigte stark schematisierte Querschnitt eines Thunfisches zeigt die Muskelstränge 15, die die stark durchbluteten roten Gewebestrukturen 16 roten Muskelfleisches umschließen. Die roten Gewebestrukturen 16 umschließen wiederum die Wirbelsäule 17. Zwischen der Wirbelsäule 17 und der Bauchhöhle 18 ist ein großes Blutgefäß 19 angeordnet, dass u.a. für die starke Durchblutung des roten Muskelfleischs verantwortlich ist. Die Muskelstränge 15 sowie das rote Muskelfleisch sind transluzente Gewebe. Die Muskelstränge 15 und die roten Gewebestrukturen 16 mit dem roten Muskelfleisch weisen verschiedene Opazitäten auf, d.h. ein jeweils unterschiedliches Lichtdurchlässigkeitsvermögen. Aufgrund der unterschiedlichen Opazitäten erscheint die Lichtlinie an der Oberfläche des Produkts 12 unterschiedlich breit. Die Detektionseinrichtung 14 ist zur Auswertung der mittels des optischen Sensors aufgenommenen Bilder ausgebildet und eingerichtet, indem insbesondere die Breite der Lichtlinien zur Erkennung des roten Muskelfleischs ausgewertet wird. Bevorzugt sind die Lichtquelle 13 und das optische Sensormittel voneinander beabstandet angeordnet, d.h. die Lichtquelle 13 und das optische Sensormittel bezüglich der Förderrichtung F in einem Abstand zueinander angeordnet, um beispielsweise die Höhenkontur des Produktes 12 zu ermitteln.

Die Detektionseinrichtung 14 - wie in der Fig. 1 gezeigt - ist derart angeordnet, dass die von dem Produkt 12 reflektierten Lichtanteile 32 unter einem Betrachtungswinkel β kleiner 90° gegenüber der Förderrichtung F aufgenommen werden. Der Betrachtungswinkel β bezeichnet hierbei den Winkel zwischen der Förderrichtung F und den reflektierten Lichtanteilen 32. Mit anderen Worten sind die Lichtquelle 13 und die Detektionseinrichtung 14 derart angeordnet, dass sowohl die Beleuchtung des Produktes 12 mittels des flächigen Lichtfeldes 31 als auch die Aufnahme der von dem Produkt 12 reflektierten Lichtanteile 32 unter Schrägstellung erfolgt.

Die Lichtquelle 13 und die Detektionseinrichtung 14 sind ferner derart angeordnet, dass der Lichtfeldwinkel a größer als der Betrachtungswinkel β ist.

Die Winkeldifferenz δ zwischen dem Lichtfeldwinkel a und dem Betrachtungswinkel β, bezeichnet den Winkel zwischen dem auf das Produkt 12 fallenden flächigen Lichtfeld 31 und den reflektierten Lichtanteilen 32.

Die Detektionseinrichtung 14 ist der Lichtquelle 13 in Förderrichtung F nachgeordnet angeordnet. Mit anderen Worten sind die Detektionseinrichtung 14 und die Lichtquelle 13 derart angeordnet, dass die Produkte 12 entgegen der Förderrichtung F schräg geneigt beleuchtet werden und die reflektierten Lichtanteile 32 zumindest eine Teilkomponente aufweisen, die ebenfalls in die Förderrichtung F zeigt.

Weiter bevorzugt ist die Lichtquelle 13 als Linienlaser ausgebildet. Vorzugsweise kommen Linienlaser mit einer Leistung von 5 bis zu 500 mW zum Einsatz. Alternativ ist die Lichtquelle 13 eine konventionelle Lichtquelle mit entsprechend hohem Lichtstrom und einer angepassten Optik zur Erzeugung des zuvor beschriebenen flächigen Lichtfeldes 31 sowie der auf dem Produkt 12 verlaufenden Lichtlinie.

Erfindungsgemäß weist die Lichtquelle 13 eine Wellenlänge im Bereich zwischen 650 und 900 nm auf, und ist damit optimal auf die Absorbtionseigenschaften von stark durchbluteten Geweben, wie den roten Gewebestrukturen 16, angepasst.

Vorteilhafter Weise ist die Detektionseinrichtung 14 zum Aufnehmen mehrerer Einzelbilder von dem Produkt 12 ausgebildet und eingerichtet. Anders ausgedrückt, ist die Detektionseinrichtung 14 derart eingerichtet, dass von dem Produkt 12 mehrere Einzelbilder erstellt werden, die eine Vielzahl von Segmenten des kontinuierlich geförderten Produkts 12 darstellen.

Weiter bevorzugt umfasst die Detektionseinrichtung 14 ein Auswertemittel, das zur Auswertung er aufgenommenen Einzelbilder ausgebildet und eingerichtet ist. Hierzu ist das Auswertemittel ferner zum Bestimmen von Grenzlinien zwischen den Bereichen der roten Gewebestrukturen 16, insbesondere des roten Muskelfleisches und den übrigen Produktbereichen 33, beispielsweise den Muskelsträngen 15, auf Basis der Einzelbilder ausgebildet und eingerichtet.

Vorzugweise ist das Auswertemittel zum Ermitteln von Linien gleicher Lichtintensität in den Einzelbildern zur Bestimmung der Grenzlinien ausgebildet und eingerichtet. Die Grenzlinien definieren den Übergang zwischen den roten Gewebestrukturen 16 und den übrigen Gewebeteilen, insbesondere der Muskelstränge 15.

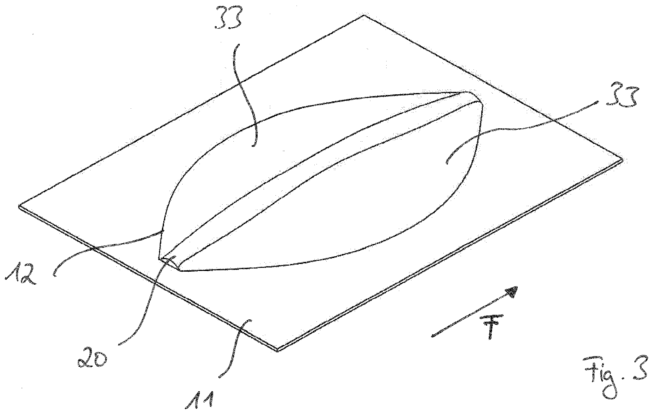

In der Figur 3 ist schematisch ein Thunfischfilet einschließlich eines Teils der Fördereinrichtung 11 in perspektivischer Darstellung gezeigt. Der zuvor genannte Bereich der roten Gewebestrukturen 16 ist als ein Streifen 20 roten Muskelfleisches der Figur 3 zu entnehmen. Das Produkt 12, beispielsweise ein Fischfilet eines Thunfisches, ist längs der Wirbelsäule 17 in die Förderrichtung F ausgerichtet und wird mittels der - in der Figur 3 nur schematisch angedeuteten - Fördereinrichtung 11 kontinuierlich gefördert.

Vorteilhafter Weise umfasst die vorliegende Erfindung auch eine Anordnung zum Lösen des Streifens 20 roter Gewebestrukturen 16 aus den Produkten 12. Die Anordnung umfasst die zuvor beschriebene Vorrichtung zur berührungslosen Erkennung der roten Gewebestrukturen 16 in dem Produkt 12 bzw. in den Produkten 12. Ferner umfasst die Anordnung eine in den Figuren nicht gezeigte Schneideinrichtung 21. Die Schneideinrichtung 21 ist zum teilweisen oder vollständigen Lösen des Streifens 20 der roten Gewebestrukturen 16 aus den Produkten 12 ausgebildet und eingerichtet. Die Schneideinrichtung 21 wird mittels einer - in der Zeichnung nicht gezeigten - Steuerungseinrichtung gesteuert. Die Steuerungseinrichtung steuert die Schneideinrichtung 21 derart, dass die mittels der erfindungsgemäßen Vorrichtung zur berührungslosen Erkennung der roten Gewebestrukturen 16 ermittelten Bereiche, insbesondere der Streifen 20, vollständig oder teilweise von den Produkten 12 gelöst wird. Die Schneideinrichtung ist wahlweise derart eingerichtet, dass entweder der Streifen 20 vollständig aus bzw. von dem Produkt 12 gelöst wird oder nur ein teilweises Lösen erfolgt, indem der Streifen 20 durch Schnitte bzw. Einschnitte in das Produkt 12 in Teilbereichen gelöst wird, jedoch nach dem Schneiden noch mit dem Produkt 12 verbunden ist, um beispielsweise mittels einer nachfolgenden manuellen Bearbeitung endgültig vom Produkt 12 gelöst zu werden.

Vorzugsweise ist die Schneideinrichtung 21 relativ zu dem Produkt 12 bewegbar ausgebildet, so dass der Abstand, insbesondere der vertikale Abstand, zwischen dem Produkt 12 und der Schneideinrichtung 21 veränderbar ausgebildet ist. Besonders bevorzugt umfasst die Schneideinrichtung 21 mindestens ein - in der Zeichnung nicht gezeigtes - Stellglied. Mittels des Stellgliedes ist die Schneideinrichtung 21 steuerbar ausgebildet, d.h. die Position der Schneideinrichtung 21 ist relativ zu dem Produkt 12 bzw. relativ zu der Fördereinrichtung 11 veränderbar eingerichtet. Mittels der Steuerungseinrichtung, wird die Schneideinrichtung in seiner Position derart gesteuert, dass die roten Gewebestrukturen bzw. der Streifen 20 teilweise oder vollständig aus dem Produkt 12 gelöst wird.

Vorteilhafter Weise ist die Schneideinrichtung 21 in der Höhe, d.h. der Abstand zwischen der jeweiligen Schneidkante und dem Produkt 12, veränderbar eingerichtet. Auf diese Weise ist die Eintauchtiefe der Schneideinrichtung 21 in das Produkt 12 zum teilweisen oder vollständigen Lösen der roten Gewebestrukturen 16 bzw. des Streifens 20 veränderbar ausgebildet.

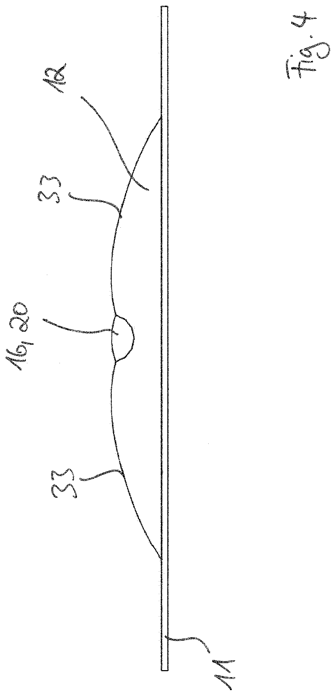

Die Figur 4 zeigt eine schematische Darstellung des in der Figur 3 gezeigten Thunfischfilets sowie des Teils der Förderreinrichtung 11 im Querschnitt. Die Förderrichtung F zeigt in der Figur 4 in die Zeichenebene hinein. Mittig ist der Bereich der roten Gewebestrukturen 16 des roten Muskelfleisches in Form des Streifens 20 zu erkennen, an den beidseitig die übrigen Produktbereiche 33 angrenzen.

Die vorliegende Erfindung umfasst auch ein Verfahren zum berührungslosen Erkennen von roten Gewebestrukturen 16 in Produkten 12 geschlachteter Tierkörper. Zur Vermeidung von Wiederholungen wird im Zusammenhang mit dem erfindungsgemäßen Verfahren vollumfänglich auf die zuvor gemachten Ausführungen bezüglich der erfindungsgemäßen Vorrichtung 10 sowie der erfindungsgemäßen Anordnung verwiesen. Die Ausführungen im Zusammenhang mit der Vorrichtung 10 und der Anordnung betreffen in analoger Weise das erfindungsgemäße Verfahren. Zur Verdeutlichung des Ablaufs des erfindungsgemäßen Verfahrens werden im Folgenden ausgewählten Aspekten des Verfahrens ergänzend beschrieben.

Das Verfahren der eingangs genannten Art umfasst die folgenden Schritte. Die Produkte 12 werden kontinuierlich in die Förderrichtung F durch einen Inspektionsbereich 30 gefördert. Der Inspektionsbereich 30 wird durch die in der Figur 1 gezeigte Lichtquelle 13 sowie die Detektionseinrichtung 14 gebildet. Der Inspektionsbereich 30 umfasst den in der Figur 1 durch die gestrichelten Linien begrenzten Erfassungsbereich der Detektionseinrichtung 14. Mittels der Lichtquelle 13 werden die Produkte 12 mittels eines flächigen Lichtfeldes 31 beaufschlagt, wobei die Lichtquelle 13 bzw. das von dieser ausgestrahlte flächige Lichtfeld 31 zur Bildung einer quer zu der Förderrichtung F verlaufenden Lichtlinie ausgebildet ist. Die von dem Produkt 12 reflektierten Lichtanteile 32 werden mittels des optischen Sensormittels der Detektionseinrichtung 14 aufgenommen. Reflektierte Lichtanteile 32 bezeichnen sowohl an der Produktoberfläche unmittelbar reflektierten Lichtanteile 32, jedoch insbesondere die Lichtanteile, die in tiefere Lagen des Produktes eindringen und dort gestreut werden. Die Beaufschlagung der Produkte 12 mit Licht erfolgt mittels der Lichtquelle 13, die als Infrarotlichtquelle ausgebildet ist. Ferner ist die Ebene des flächigen Lichtfeldes gegenüber der Fördereinrichtung 11 um einen Lichtfeldwinkel a kleiner 90° geneigt.

## Patentansprüche

1. Vorrichtung (10) zur berührungslosen Erkennung roter Gewebestrukturen (16) in Produkten (12) geschlachteter Tierkörper, umfassend eine Fördereinrichtung (11) zum kontinuierlichen Fördern der Produkte (12) in eine Förderrichtung (F), eine zur Erzeugung eines flächigen Lichtfelds (31) eingerichtete Lichtquelle (13), die zum Bilden einer quer zu der Förderrichtung (F) des Produktes (12) verlaufenden Lichtlinie aus dem flächigen Lichtfeld (31) ausgebildet und eingerichtet ist, eine Detektionseinrichtung (14) zur Erkennung der roten Gewebestrukturen (16), die mindestens ein optisches Sensormittel zur Aufnahme der von dem Produkt (12) reflektierten Lichtanteile (32) umfasst, wobei die Lichtquelle (13) als eine Infrarotlichtquelle ausgebildet ist, wobei die Lichtquelle (13) und das optische Sensormittel in Förderrichtung (F) voneinander beabstandet angeordnet sind, wobei
die Lichtquelle (13) derart angeordnet ist, dass die Ebene des flächigen Lichtfeldes (31) gegenüber der Förderrichtung (F) um einen Lichtfeldwinkel (α) kleiner 90° geneigt ist und die Detektionseinrichtung (14) derart angeordnet ist, dass die von dem Produkt (12) reflektierten Lichtanteile (32) unter einem Betrachtungswinkel (β) kleiner 90° gegenüber der Förderrichtung (F) aufgenommen werden und die Lichtquelle (13) und die Detektionseinrichtung (14) derart angeordnet sind, dass der Lichtfeldwinkel (α) größer als der Betrachtungswinkel (β) ist,
und wobei
die Lichtquelle (13) eine Wellenlänge zwischen 650 nm und 900 nm aufweist und
die Detektionseinrichtung (14) ausgebildet ist, die Linienbreite der Lichtlinie auf dem Produkt (12) auszuwerten.

2. Vorrichtung nach Anspruch 1, **dadurch gekennzeichnet, dass** die Detektionseinrichtung (14) der Lichtquelle (13) in Förderrichtung (F) nachgeordnet angeordnet ist.

3. Vorrichtung nach einem der Ansprüche 1 oder 2, **dadurch gekennzeichnet, dass** die Lichtquelle (13) als Linienlaser ausgebildet ist.

4. Vorrichtung nach einem der Ansprüche 1 bis 3, **dadurch gekennzeichnet, dass** die Detektionseinrichtung (14) zum Aufnehmen mehrerer Einzelbilder ausgebildet und eingerichtet ist.

5. Vorrichtung nach Anspruch 4, **dadurch gekennzeichnet, dass** die Detektionseinrichtung (14) ein Auswertemittel umfasst, das zum Bestimmen von Grenzlinien zwischen Bereichen der roten Gewebestrukturen (16) und den übrigen Produktbereichen (33) des Produktes (12) auf Basis der Einzelbilder ausgebildet und eingerichtet ist.

6. Vorrichtung nach Anspruch 5, **dadurch gekennzeichnet, dass** das Auswertemittel zum Ermitteln von Linien gleicher Lichtintensität in den Einzelbildern zur Bestimmung der Grenzlinien ausgebildet und eingerichtet ist.

7. Anordnung zum Lösen eines Streifens (20) roter Gewebestrukturen (16) aus Produkten (12) geschlachteter Tierkörper, umfassend eine Vorrichtung (10) zur berührungslosen Erkennung der roten Gewebestrukturen (16) in den Produkten (12), eine Schneideinrichtung (21) zum teilweisen oder vollständigen Lösen der roten Gewebestrukturen (16) aus den Produkten (12), eine Steuerungseinrichtung, die zur Steuerung der Schneideinrichtung (21) entlang der mittels der Vorrichtung (10) zur berührungslosen Erkennung der roten Gewebestrukturen (16) ermittelten Bereiche eingerichtet und ausgebildet ist, wobei die Vorrichtung (10) zur berührungslosen Erkennung der roten Gewebestrukturen (16) in den Produkten (12) nach einem der Ansprüche 1 bis 6 ausgebildet ist.

8. Anordnung nach Anspruch 7, **dadurch gekennzeichnet, dass** die Schneideinrichtung (21) relativ zu dem Produkt (12) bewegbar ausgebildet ist.

9. Anordnung nach Anspruch 8, **dadurch gekennzeichnet, dass** die Schneideinrichtung (21) mindestens ein Stellglied umfasst, mittels dessen die Schneideinrichtung (21) zum teilweisen oder vollständigen Lösen der roten Gewebestrukturen (16) aus dem Produkt (12) steuerbar eingerichtet ist.

10. Anordnung nach einem der Ansprüche 7 bis 9, **dadurch gekennzeichnet, dass** die Schneideinrichtung (21) höhenverstellbar ausgebildet und eingerichtet ist, so dass zum teilweisen oder vollständigen Lösen der roten Gewebestrukturen (16) aus dem Produkt (12) die Eintauchtiefe der Schneideinrichtung (21) in das Produkt (12) veränderbar ausgebildet ist.

11. Verfahren zum berührungslosen Erkennen roter Gewebestrukturen (16) in Produkten (12) geschlachteter Tierkörper, umfassend die Schritte:
- kontinuierliches Fördern der Produkte (12) in eine Förderrichtung (F) durch einen Inspektionsbereich (30),
- Beaufschlagen der Produkte (12) mittels eines flächigen Lichtfeldes (31) einer Lichtquelle (13) zum Bilden einer quer zu der Förderrichtung (F) des Produkts (12) verlaufenden Lichtlinie,
- Aufnehmen der von dem Produkt (12) reflektierten Lichtanteile (32) mittels eines optischen Sensormittels einer Detektionseinrichtung (14), wobei die Lichtquelle (13) und das optische Sensormittel in Förderrichtung (F) voneinander beabstandet angeordnet sind, wobei
die Lichtquelle (13) als Infrarotlichtquelle ausgebildet ist und die Ebene des flächigen Lichtfeldes (31) gegenüber der Förderrichtung (F) um einen Lichtfeldwinkel (α) kleiner 90° geneigt ist und das Aufnehmen der von dem Produkt (12) reflektierten Lichtanteile (32) unter einem Betrachtungswinkel (β) kleiner 90° gegenüber der Förderrichtung (F) erfolgt, wobei der Lichtfeldwinkel (α) größer als der Betrachtungswinkel (β) ist, wobei
die Lichtquelle (13) eine Wellenlänge zwischen 650 nm und 900 nm aufweist und
die Detektionseinrichtung (14) ausgebildet ist, die Linienbreite der Lichtlinie auf dem Produkt (12) auszuwerten.

12. Verfahren nach Anspruch 11, **dadurch gekennzeichnet, dass** mehrere Einzelbilder mittels der Detektionseinrichtung (14) von jedem der Produkte (12) aufgenommen werden.

13. Verfahren nach Anspruch 12, **dadurch gekennzeichnet, dass** auf Basis der Einzelbilder mittels eines Auswertemittels Grenzlinien zwischen Bereichen der roten Gewebestrukturen (16) und den übrigen Produktbereichen (33) des Produktes (12) bestimmt werden.

14. Verfahren nach Anspruch 13, **dadurch gekennzeichnet, dass** Linien gleicher Lichtintensität in den Einzelbildern zur Bestimmung der Grenzlinien mittels des Auswertemittels bestimmt werden.

## Claims

1. Apparatus (10) for non-contact identifying of red tissue structures (16) in products (12) of slaughtered animal bodies, comprising a conveying device (11) for continuous conveyance of the products (12) in a conveying direction (F), a light source (13) configured to generate a flat light field (31) which is designed and adapted to form a light line running transversely to the conveying direction (F) of the product (12) from the flat light field (31), a detecting device (14) for identifying the red tissue structures (16) which comprises at least one optical sensor means for recording the portions of light (32) reflected by the product (12), wherein the light source (13) is configured as an infrared light source, wherein
the light source (13) and the optical sensor means are arranged spaced apart in the conveying direction (F), wherein
the light source (13) is arranged such that the plane of the flat light field (31) is tilted relative to the conveying direction (F) by a light field angle (α) of less than 90°, and
the detecting device (14) is arranged such that the light portions (32) reflected by the product (12) are recorded at a viewing angle (β) of less than 90° relative to the conveying direction (F), and
the light source (13) and detecting device (14) are arranged such that the light field angle (α) is greater than the viewing angle (β), and wherein
the light source (13) has a wavelength between 650 nm and 900 nm and
the detecting device (14) is designed to evaluate the line width of the light line on the product (12).

2. Apparatus according to claim 1, **characterised in that** the detecting device (14) is arranged downstream of the light source (13) in the conveying direction (F).

3. Apparatus according to claim 1 or 2, **characterised in that** the light source (13) is designed as a linear laser.

4. Apparatus according to any one of claims 1 to 3, **characterised in that** the detecting device (14) is designed and adapted to record a plurality of individual images.

5. Apparatus according to claim 4, **characterised in that** the detecting device (14) comprises an analysis means which is designed and adapted to determine border lines between areas of the red tissue structures (16) and the other product areas (33) of the product (12) on the basis of the individual images.

6. Apparatus according to claim 5, **characterised in that** the analysis means is designed and adapted to detect lines of the same light intensity in the individual images in order to determine the border lines.

7. Arrangement for removing a strip (20) of red tissue structures (16) from products (12) of slaughtered animal bodies, comprising an apparatus (10) for non-contact identifying of red tissue structures (16) in the products (12), a cutting device (21) for partial or complete removal of the red tissue structures (16) from the products (12), a control device adapted and designed to control the cutting device (21) along the regions determined by means of the apparatus (10) for non-contact identifying of red tissue structures (16), wherein the apparatus (10) for non-contact identifying of red tissue structures (16) in the products (12) is designed according to any one of claims 1 to 6.

8. Arrangement according to claim 7, **characterised in that** the cutting device (21) is designed moveably relative to the product (12).

9. Arrangement according to claim 8, **characterised in that** the cutting device (21) comprises at least one actuator, by means of which the cutting device (21) is adapted controllably for partial or complete removal of the red tissue structures (16) from the product (12).

10. Arrangement according to any one of claims 7 to 9, **characterised in that** the cutting device (21) is designed and adapted height-adjustably so that the immersion depth of the cutting device (21) in the product (12) is designed variably for partial or complete removal of the red tissue structures (16) from the product (12).

11. Method for non-contact identifying of red tissue structures (16) in products (12) of slaughtered animal bodies, comprising the steps:
- continuous transport of the products (12) through an inspection area (30) in a conveying direction (F),
- illumination of the products (12) by means of a flat light field (31) of a light source (13) to form a light line running transversely to the conveying direction (F) of the product (12),
- recording of the portions of light (32) reflected by the products (12) by means of an optical sensor means of a detecting device (14), wherein the light source (13) and the optical sensor means are arranged spaced apart in the conveying direction (F), wherein
the light source (13) is designed as an infrared light source and the plane of the flat light field (31) relative to the conveying direction (F) is tilted by a light field angle (α) of less than 90°, and the portions of light (32) reflected by the product (12) are recorded at a viewing angle (β) of less than 90° relative to the conveying direction (F), wherein the light field angle (α) is greater than the viewing angle (β), wherein
the light source (13) has a wavelength between 650 nm and 900 nm and
the detecting device (14) is designed to evaluate the line width of the light line on the product (12).

12. Method according to claim 11, **characterised in that** a plurality individual images is recorded by means of the detecting device (14) of each of the products (12).

13. Method according to claim 12, **characterised in that**, on the basis of the individual images, by means of an analysis means, border lines are determined between areas of red tissue structures (16) and the other product areas (33) of the product (12).

14. Method according to claim 13, **characterised in that** lines of the same light intensity are determined in the individual images for the determination of the border lines by means of the analysis means.

## Revendications

1. Dispositif (10) destiné à identifier sans contact des structures tissulaires rouges (16) dans des produits (12) de carcasses d'animaux abattus, comprenant un dispositif de convoyage (11) destiné à convoyer les produits (12) en continu dans une direction de convoyage (F), une source de lumière (13) configurée pour générer un champ lumineux plat (31), qui est conçue et configurée pour former une ligne lumineuse à partir du champ lumineux plat (31), s'étendant transversalement à la direction de convoyage (F) du produit (12), un dispositif de détection (14) destiné à identifier les structures tissulaires rouges (16), qui comprend au moins un moyen capteur optique destiné à recevoir les parts de lumière (32) réfléchies par le produit (12), dans lequel la source de lumière (13) est conçue sous forme de source de lumière infrarouge, dans lequel
la source de lumière (13) et le moyen capteur optique sont agencés dans la direction de convoyage (F) à distance l'une de l'autre, dans lequel
la source de lumière (13) est agencée de telle façon que le plan du champ lumineux plat (31) est incliné par rapport à la direction de convoyage (F) sous un angle de champ lumineux (α) inférieur à 90° et
le dispositif de détection (14) est agencé de telle façon que les parts de lumière (32) réfléchies par le produit (12) sont reçues sous un angle de vue (β) inférieur à 90° par rapport à la direction de convoyage (F) et
la source de lumière (13) et le dispositif de détection (14) sont agencés de telle façon que l'angle de champ lumineux (α) est supérieur à l'angle de vue (β), et dans lequel
la source de lumière (13) présente une longueur d'onde entre 650 nm et 900 nm
le dispositif de détection (14) est conçue pour évaluer la largeur de ligne de la ligne lumineuse sur le produit (12).

2. Dispositif selon la revendication 1, **caractérisé en ce que** le dispositif de détection (14) est agencé en aval de la source de lumière (13) dans la direction de convoyage (F).

3. Dispositif selon l'une quelconque des revendications 1 ou 2, **caractérisé en ce que** la source de lumière (13) est conçue sous forme de laser linéaire.

4. Dispositif selon l'une quelconque des revendications 1 à 3, **caractérisé en ce que** le dispositif de détection (14) est conçu et configuré pour recevoir une pluralité d'images individuelles.

5. Dispositif selon la revendication 4, **caractérisé en ce que** le dispositif de détection (14) comprend un moyen d'évaluation qui est conçu et configuré pour définir des lignes frontières entre des zones des structures tissulaires rouges (16) et les zones de produit restantes (33) du produit (12) sur la base des images individuelles.

6. Dispositif selon la revendication 5, **caractérisé en ce que** le moyen d'évaluation est conçu et configuré pour déterminer des lignes de même intensité lumineuse dans les images individuelles pour définir les lignes frontières.

7. Agencement destiné à détacher une bande (20) de structures tissulaires rouges (16) hors de produits (12) de carcasses d'animaux abattus, comprenant un dispositif (10) destiné à identifier sans contact les structures tissulaires rouges (16) dans les produits (12), un dispositif de coupe (21) destiné à détacher partiellement ou entièrement les structures tissulaires rouges (16) hors des produits (12), un dispositif de commande qui est conçu et configuré pour commander le dispositif de coupe (21) le long des zones déterminées au moyen du dispositif (10) destiné à identifier sans contact les structures tissulaires rouges (16), dans lequel le dispositif (10) destiné à identifier sans contact les structures tissulaires rouges (16) dans les produits (12) est conçu selon l'une quelconque des revendications 1 à 6.

8. Agencement selon la revendication 7, **caractérisé en ce que** le dispositif de coupe (21) est conçu de manière mobile relativement au produit (12).

9. Agencement selon la revendication 8, **caractérisé en ce que** le dispositif de coupe (21) comprend au moins un élément de positionnement au moyen duquel le dispositif de coupe (21) est configuré de manière à pouvoir être commandé pour détacher partiellement ou entièrement les structures tissulaires rouges (16) hors du produit (12).

10. Agencement selon l'une quelconque des revendications 7 à 9, **caractérisé en ce que** le dispositif de coupe (21) est conçu et configuré de manière à pouvoir être ajusté en hauteur de sorte que la profondeur d'immersion du dispositif de coupe (21) dans le produit (12) est conçue de manière à pouvoir être modifiée pour détacher partiellement ou entièrement les structures tissulaires rouges (16) hors du produit (12).

11. Procédé destiné à identifier sans contact des structures tissulaires rouges (16) dans des produits (12) de carcasses d'animaux abattus, comprenant les étapes consistant à :
- convoyer les produits (12) en continu dans une direction de convoyage (F) à travers une zone d'inspection (30),
- appliquer aux produits (12) une source de lumière (13) d'un champ lumineux plat (31) pour former une ligne lumineuse s'étendant transversalement à la direction de convoyage (F) du produit (12),
- recevoir les parties de lumière (32) réfléchies par le produit (12) via un moyen capteur optique d'un dispositif de détection (14), dans lequel la source de lumière (13) et le moyen capteur optique sont agencés dans la direction de convoyage (F) à distance l'une de l'autre, dans lequel
la source de lumière (13) est conçue sous forme de source de lumière infrarouge et le plan du champ lumineux plat (31) est incliné par rapport à la direction de convoyage (F) sous un angle de champ lumineux (α) inférieur à 90° et la réception des parts de lumière (32) réfléchies par le produit (12) s'effectue sous un angle de vue (β) inférieur à 90° par rapport à la direction de convoyage (F), dans lequel l'angle de champ lumineux (α) est supérieur à l'angle de vue (β), dans lequel
la source de lumière (13) présente une longueur d'onde entre 650 nm et 900 nm
le dispositif de détection (14) est conçue pour évaluer la largeur de ligne de la ligne lumineuse sur le produit (12).

12. Procédé selon la revendication 11, **caractérisé en ce qu'**une pluralité d'images individuelles de chacun des produits (12) sont reçues au moyen du dispositif de détection (14).

13. Procédé selon la revendication 12, **caractérisé en ce que**, sur la base des images individuelles, des lignes frontières sont définies au moyen d'un dispositif d'évaluation entre des zones des structures tissulaires rouges (16) et les zones de produit restantes (33) du produit (12).

14. Procédé selon la revendication 13, **caractérisé en ce que** des lignes de même intensité lumineuse sont définies dans les images individuelles pour déterminer les lignes frontières via le moyen d'évaluation.
